# EUROPÄISCHE PATENTANMELDUNG

(11) **EP 3 333 146 A1**
(43) Veröffentlichungstag der Anmeldung: **13.06.2018**
(21) Anmeldenummer: 16202819.5
(22) Anmeldetag: 08.12.2016
(51) Int. Cl.: C07C 43/04, C07C 41/06, C07C 41/58

(54) **KOSTENGÜNSTIGE HERSTELLUNG VON HOCHREINEM METHYL-TERT.-BUTYLETHER**

(71) Anmelder: Evonik Degussa GmbH, 45128 Essen (DE)
(72) Erfinder: SCHALLENBERG, Jörg, 46286 Dorsten (DE)

(57) **Zusammenfassung**

Der Erfindung liegt die Aufgabe zu Grunde, ein Verfahren zur Herstellung von hochreinem Methyl-tert.-butylether anzugeben, welches geringere Produktionskosten verursacht. Das Verfahren soll als Ausgangsgemisch insbesondere MTBE in Kraftstoff-Qualität nutzen, aber auch mit stärker verunreinigtem MTBE, besser noch mit rohem Reaktionsgemisch beschickt werden können. Das Verfahren soll solche Verunreinigungen effizient entfernen, die typischerweise in dem Produktionsprozess von Methyl-tert.-butylether vorkommen und schwierig abzutrennen sind wie Isobuten, weitere Kohlenwasserstoffe mit vier oder fünf Kohlenstoffatomen, tert.-Butanol, Methanol, Methyl-sec.-butylether oder Wasser. Das Verfahren soll im industriellen Maßstab ausführbar sein und auf bewährter Destillationstechnologie beruhen. Eine Grundidee besteht darin, die Synthese und Aufreinigung dergestalt aneinander anzupassen, dass in der Synthese nicht mehr Methanol eingesetzt wird, als in dem Azeotrop aus Methanol und den C4-Kohlenwasserstoffen aus der den Syntheseprozess abschließenden Kolonne austragbar ist.

## Beschreibung

Methyl-tert.-butylether (IUPAC Name: 2-methoxy-2-methylpropane; Summenformel: C₅H₁₂O, international gebräuchliches Akronym: MTBE) ist eine Chemikalie, die grundsätzlich in zwei Qualitäten gehandelt wird:
Die weitaus größte Handels- und Produktionsmenge macht Kraftstoff-MTBE aus, auch genannt MTBE in Kraftstoffqualität, Benzin-MTBE oder technisches MTBE. Die Anmelderin bietet Kraftstoff-MTBE unter der Marke DRIVERON® an. Als klopfhemmender Zusatz in Kraftstoffen für Ottomotoren bewirkt MTBE eine wesentliche Erhöhung der Oktanzahl und trägt als Sauerstoff enthaltende Komponente zur Optimierung der motorischen Verbrennung bei. Aufgrund dieser Verwendung wird Kraftstoff-MTBE weltweit in großen Mengen produziert; im Jahr 2005 lag die Weltproduktionsmenge für Kraftstoff-MTBE bei 18*10⁹ kg.
In deutlich geringeren Mengen gehandelt wird hochreiner MTBE, auch genannt MTBE/S oder HP MTBE. Die Anmelderin vertreibt hochreinen MTBE unter der Marke DRIVERON®S.

Hochreiner MTBE ist eine Feinchemikalie, die vor allem als Löse- und Extraktionsmittel Verwendung findet, etwa in der pharmazeutischen Industrie sowie bei der Herstellung von Insektiziden und Pestiziden. Hochreiner MTBE ist deutlich teurer als Kraftstoff-MTBE.

Die Zusammensetzung von Kraftstoff-MTBE und hochreinem MTBE hängt von der Herstellung ab. Typische Produktspezifikationen sind in Tabelle 1 dargelegt.

**Tabelle 1: Typische Zusammensetzungen von Kraftstoff-MTBE und hochreinem MTBE (Angaben in Gewichtsanteilen)**

| **Komponente** | **Kraftstoff-MBTE** | **hochreiner MTBE** |
|---|---|---|
| MTBE (chemische Reinsubstanz) | mindestens 98 % | mindestens 99.9 % |
| Kohlenwasserstoffe mit bis zu 8 Kohlenstoffatomen | weniger als 0.5 % | weniger als 0.02 % |
| tert.-Butanol | weniger als 1 % | weniger als 0.025 % |
| Methanol | weniger als 1 % | weniger als 0.01 % |
| Wasser | weniger als 500 ppm | weniger als 100 ppm |

Eine umfassende Darstellung der Herstellung von MTBE in Kraftstoffqualität bieten
Markus Winterberg, Ekkehard Schulte-Körne, Udo Peters and Franz Nierlich: Methyl Tert-Butyl Ether. Ullmann's Encyclopedia of Industrial Chemistry. Article first published online: 15 APR 2010 | DOI: 10.1002/14356007.a16_543.pub2

Demnach wird MTBE durch Reaktion des C₄-Olefins Isobuten mit Methanol synthetisiert und das erhaltene, rohe Reaktionsgemisch anschließend zu Kraftstoff-MTBE destillativ aufgereinigt. Die destillative Aufreinigung des Reaktionsgemisches zu Kraftstoff-MTBE erfolgt bei neueren Produktionsanlagen häufig mit Hilfe einer Reaktivdestillationskolonne.

Die weitergehende Aufreinigung von Kraftstoff-MTBE zu hochreinem MTBE ist nicht trivial: So enthält Kraftstoff-MTBE auf Grund seines Syntheseweges und der eingesetzten Rohstoffe meist tert.-Butanol (TBA), Methanol (MeOH) und Methyl-sec.-butylether (MSBE) als Verunreinigungen. Diese Substanzen bilden mit dem MTBE ein schwierig zu brechendes Azeotrop bzw. im Fall von MSBE ein sehr eng siedendes System, sodass die destillative Herstellung von hochreinem MTBE, welcher sehr geringe Restanteile TBA, MeOH und MSBE enthält, sehr aufwändig ist.

Auch Restanteile von Isobuten sind problematisch: Isobuten ist neben Methanol das Edukt bei der Synthese von MTBE. Wenn die Reaktion nicht vollständig erfolgt, bleibt nicht umgesetztes Isobuten zurück. Dies ist zu vermeiden, da Isobuten über Diisobuten zu Polyisobuten weiterreagieren kann und dann einen zähen Film auf den Apparaten bildet, ähnlich wie Kaugummi. Da es sich bei der MTBE-Synthese um eine Gleichgewichtsreaktion handelt, wird im technischen Prozess zur Herstellung von Kraftstoff-MTBE mit einem Überschuss an Methanol gearbeitet. Dies senkt die Gefahr einer Verklebung mit Polyisobuten, erhöht aber die Verunreinigung mit Methanol. Insoweit besteht ein Zielkonflikt in der Reinheit des MTBE.

Neben Isobuten können auch andere Kohlenwasserstoffe mit vier Kohlenstoffatomen als Verunreinigungen in technischem MTBE enthalten sein, nämlich 1,3-Butadien, n-Buten, Isobutan und n-Butan. n-Buten ist eine Sammelbezeichnung von 1-Buten, cis-2-Buten und trans-2-Buten. Diese Stoffe kommen in den meisten für die MTBE-Synthese genutzten, Isobuten-haltigen Rohstoffen vor, werden jedoch zur anderweitigen Nutzung vor oder nach einer MTBE-Synthese abgetrennt. Je nachdem, wie gut die Abtrennung gelingt, liegen noch entsprechende Rückstände als Verunreinigungen im MTBE vor. Kohlenwasserstoffe mit vier Kohlenstoffatomen werden einzeln oder im Gemisch als C₄ bezeichnet.

Als Verunreinigungen ebenso abzutrennen sind etwaige Kohlenwasserstoffe mit fünf Kohlenstoffatomen, nämlich die Isopren, Pentane und Pentene. Sie sind häufig in industriellen C₄-Einsatzrohstoffen als Nebenbestandteil enthalten und werden in der Fachwelt einzeln oder gemeinsam als C₅ bezeichnet.
Noch ein paar Worte zum Wasser: In den allermeisten Fällen sind die für die MTBE-Synthese eingesetzten C₄-Kohlenwasserstoffgemische mit Wasser gesättigt. Das enthaltene Wasser reagiert parallel zur MTBE-Synthese mit dem Isobuten zu TBA. Da MTBE hydrophil ist, gelangt auch durch unsachgemäße Lagerung Wasser aus feuchter Umgebungsluft hinein. Die von den Kunden tolerierten Restgehalte an Wasser in hochreinem MTBE liegen im zweistelligen ppm-Bereich. Sofern der Produzent unerwünschten Wassereintrag nicht unterbinden kann, muss er es bis zur Toleranzgrenze wieder entfernen.

Der letzte Aufreinigungsschritt von technischem MTBE zu hochreinem Methyl-tert.-butylether ist also mit großen Schwierigkeiten verbunden. Die Reduktion des Gehalts an schwierig zu entfernenden Komponenten wie Isobuten, weitere Kohlenwasserstoffe mit vier und fünf Kohlenstoffatomen, tert.-Butanol, Methanol, Methyl-sec.-butylether oder Wasser um ein paar minimale Gew.-% erfordert einen überproportional großen Aufwand. Deswegen bestimmt der Restgehalt dieser Komponenten im hochreinen MTBE maßgeblich den Preis dieser Feinchemikalie.

Ein gangbarer Weg zur destillativen Herstellung von hochreinem MTBE ist in US7304188B2 geschildert. Dabei wird technischer MTBE in einem Zwei-Kolonnen-System destillativ gereinigt, wobei hochreiner MTBE in einer Mittelfraktion anfällt. Dieses Verfahren ermöglicht es Kraftstoff-MTBE (wie in Tabelle 1 links angegeben) in hochreinen MTBE gemäß der rechten Spalte der Tabelle 1 zu überführen. Da neben dem zwei-Kolonnensystem noch eine weitere Kolonne notwendig ist, um das rohe Reaktionsgemisch zu technischem MTBE aufzuarbeiten, erfolgt die Aufreinigung insgesamt gesehen in drei Stufen.

Ein erster Nachteil dieses Verfahrens ist, dass insgesamt drei Destillationsstufen durchlaufen werden. Dies ist ein großer apparativer Aufwand, welcher hohe Produktionskosten verursacht. Ein zweiter Nachteil dieses Verfahren ist, dass vom Zwei-Kolonnen-Systems ein vergleichsweise großer Leichtsieder-Massenstrom abgezogen wird. Da die Leichtsieder über Kopf gehen, müssen diese verdampft werden; daraus resultiert ein hoher Energiebedarf.

Die Effizienz dieses Prozesses, gemessen an der Menge des hochreinen Ethers erhalten pro Menge des eingesetzten, verunreinigten Ethers, ist mithin gering. Dies ist ein weiterer Grund dafür, warum das in US7304188B2 beschriebene Verfahren die Herstellung von hochreinem MTBE nur unter hohen Kosten ermöglicht.

Gleichwohl ist das in US7304188B2 beschriebene destillative Verfahren industriell praktikabel; alternative Wege zur Herstellung von hochreinem MTBE sind allenfalls im Labormaßstab ausführbar.

In Hinblick auf diesen Stand der Technik liegt der Erfindung die Aufgabe zu Grunde, ein Verfahren zur Herstellung von hochreinem Methyl-tert.-butylether anzugeben, welches geringere Produktionskosten verursacht. Das Verfahren soll als Ausgangsgemisch insbesondere MTBE in Kraftstoff-Qualität nutzen, aber auch mit stärker verunreinigtem MTBE, besser noch mit rohem Reaktionsgemisch beschickt werden können. Mit dem Verfahren sollen insbesondere solche Verunreinigungen effizient entfernt werden können, die typischerweise in dem Produktionsprozess von Methyl-tert.-butylether vorkommen und schwierig abzutrennen sind wie Isobuten, weitere Kohlenwasserstoffe mit vier oder fünf Kohlenstoffatome, tert.-Butanol, Methanol, Methyl-sec.-butylether oder Wasser. Das Verfahren soll im industriellen Maßstab ausführbar sein und auf bewährter Destillationstechnologie beruhen.

Nun wurde gefunden, dass eine einfache Fraktionierung ausreichend ist, bei der hochreiner Methyl-tert.-butylether als Kopfprodukt abgenommen wird. Dieser Befund ist überraschend, denn bisher waren MTBE-Fachleute davon ausgegangen, dass hochreiner MTBE nur als Mittelfraktion zu gewinnen sei. Um es hingegen über Kopf abnehmen zu können, ist es erforderlich, dass das Sumpfprodukt der (Reaktivdestillations-)Kolonne, welche das rohe Reaktionsgemisch der MTBE-Synthese aufarbeitet, nur geringe Mengen Leichtsieder (Kohlenwasserstoffe mit vier Kohlenstoffatome, Methanol, Wasser) enthält. Die Funktion der Leichtsiederabtrennung wird somit in die den Syntheseprozess abschließende (Reaktivdestillations-)Kolonne integriert. Die Leichtsieder werden also erfindungsgemäß über Kopf der den Syntheseprozess abschließende Kolonne abgetrennt. Diese Funktion kann in der den Syntheseprozess abschließende Kolonne durchgeführt werden, wenn
- bei einer bestehenden Anlage das Rücklaufverhältnis dieser Kolonne erhöht wird;
- bei der Planung/Bau einer Anlage die Stufenzahl erhöht wird, bzw. die abschließende Kolonne den Anforderungen entsprechend ausgelegt wird,
- oder wenn die Kapazität der Anlage zeitweise reduziert wird, um das hochreine MTBE zu produzieren (Kampagnen-Fahrweise).

Gegenüber einem herkömmlichen Zwei-Kolonnen-System zur Aufreinigung von technischem MTBE, bei welchem der hochreine MTBE in der Mittelfraktion anfällt, wird mithin eine Kolonne eingespart, da die ohnehin vorhandene, den Syntheseprozess abschließende Kolonne die Funktion der Leichtsiederabtrennung von der ersten Kolonne eines klassischen Zwei-Kolonnen-Systems mit übernimmt.

Um eine ausreichende Abtrennung des Methanols in der den Syntheseprozess abschließenden Kolonne sicherzustellen, ist dort allerdings eine Überdosierung des Methanols im Verhältnis zum Isobuten zu begrenzen. Um das Verfahren wie beschrieben durchführen zu können, darf am Eingang der den Syntheseprozess abschließenden Kolonne nur so viel Methanol enthalten sein, wie im Verhältnis zu den C₄-Komponenten als Leichtsiederfraktion abgeführt werden kann. Die abführbare Methanol-Menge ist abhängig von der Zusammensetzung, der Menge der C₄-Komponenten im Verhältnis zum MTBE und vom Druck des Trennapparates. Diese Menge Methanol, die mit den Kohlenwasserstoffen mit vier Kohlenstoffatomen über Kopf abgezogen werden kann, liegen thermodynamische physikalische Gesetzmäßigkeiten zu Grunde. Methanol und C4 bilden ein Azeotrop. Es ist deswegen nicht möglich die maximale Menge bzw. Konzentration von Methanol in dem über Kopf abzutrennenden Strom durch Erhöhung der Trennleistung (etwa durch Erhöhung des Rücklaufs oder das Vorsehen von mehr Trennstufen) über das thermodynamische Gleichgewicht hinaus abzutrennen.

Die bereits mehrfach erwähnte, den Syntheseprozess abschließende Kolonne ist der Apparat innerhalb der Produktionsanlage, der eine thermische Trennoperation ausführt und von dem aus stromabwärts gesehen keine Synthese von MTBE mehr vorgesehen ist.

Im einfachsten Fall ist dies eine Destillationskolonne, die stromabwärts unmittelbar hinter dem Reaktor angeordnet ist, in welchem die MTBE-Synthese erfolgt. Die Reaktion erfolgt demnach stromaufwärts vor der den Syntheseprozess abschließende Kolonne.

In Prozessen, in welchem die MTBE-Synthese mehrstufig mit Zwischenabtrennung erfolgt, ist die den Syntheseprozess abschließende Kolonne die erste Kolonne hinter der letzten Synthesestufe (stromabwärts gesehen). Auch hier erfolgt die Reaktion stromaufwärts vor der den Syntheseprozess abschließende Kolonne, allerdings in mehreren in Reihe geschaltete Reaktoren. Eine zur Zwischenabtrennung zwischen den einzelnen Synthesestufen (Reaktoren) angeordnete Kolonne ist demnach keine den Syntheseprozess abschließende Kolonne im Sinne der Erfindung.

Moderne MTBE-Anlagen arbeiten mit einem Synthesereaktor und einer stromabwärts dahinter angeordneten Reaktivdestillationskolonne. Die Reaktivdestillationskolonne erfüllt dabei zwei Funktionen, nämlich die eines weiteren Syntheseschrittes und zugleich die destillative Abtrennung des technischen MTBE aus dem Reaktionsgemisch. Bei einen solchen Prozesslayout ist die Reaktivdestillationskolonne die den Syntheseprozess abschließende Kolonne. In ihr selbst findet zwar noch eine MTBE-Synthese statt, stromabwärts von ihr gesehen aber nicht mehr.

In allen Fällen markiert die den Syntheseprozess abschließende Kolonne zugleich den Beginn des auf den Syntheseprozess folgenden Reinigungsprozesses, im Zuge dessen Verunreinigungen aus dem rohen Reaktionsgemisch entfernt werden.

Die gestellte Aufgabe wird konkret gelöst durch ein Verfahren zur Herstellung von hochreinem Methyl-tert.-butylether, bei welchem in einem Syntheseprozess Isobuten mit Methanol zu einem Reaktionsgemisch umgesetzt wird, welches Methyl-tert.-butylether und als Verunreinigungen zumindest Kohlenwasserstoffe mit vier Kohlenstoffatomen, Methanol und höher als Methyl-tert.-butylether siedende Substanzen enthält, und bei welchem sodann unter nicht alleiniger Verwendung einer den Syntheseprozess abschließenden Kolonne in einem Reinigungsprozess die Verunreinigungen des Reaktionsgemisches nahezu vollständig destillativ entfernt werden, wodurch hochreiner Methyl-tert.-butylether erhalten wird, bei welchem der Syntheseprozess und Reinigungsprozess so aufeinander abgestimmt sind, dass die Menge an Methanol in dem Reaktionsgemisch kleiner ist als die Menge an Methanol in einem Azeotrop, welches aus Methanol und Kohlenwasserstoffen mit vier Kohlenstoffatomen in der den Syntheseprozess abschließenden Kolonne gebildet und über deren Kopf abgezogen wird, und bei welchem das Sumpfprodukt der den Syntheseprozess abschließenden Kolonne in einer zweiten, im Reinigungsprozess verwendeten Kolonne destillativ getrennt wird in hochreinen Methyl-tert.-butylether, welcher vom Kopf der zweiten im Reinigungsprozess verwendeten Kolonne abgezogen wird, und in eine Fraktion enthaltend höher als Methyl-tert.-butylether siedende Substanzen.

Ein solches Verfahren ist ein erster Gegenstand der Erfindung.

Eine Grundidee der Erfindung besteht darin, die Synthese und Aufreinigung dergestalt aneinander anzupassen, dass in der Synthese nicht mehr Methanol eingesetzt wird, als in dem Azeotrop aus Methanol und den C₄-Kohlenwasserstoffen aus der den Syntheseprozess abschließenden Kolonne austragbar ist. Auf diese Weise ist es möglich, in der nächsten Trennstufe unmittelbar hochreinen MTBE über Kopf abzuziehen. Gegenüber dem im US7304188B2 geschilderten Verfahren kann mithin eine Trennstufe eingespart werden. Die Herstellkosten des hochreinen MTBE werden dadurch geringer.

Wird das erfindungsgemäße Verfahren unmittelbar in Kombination mit einer MTBE-Synthese betrieben, kann die notwendige Abstimmung zwischen Synthese und Destillation mit Hilfe der üblichen Prozessleittechnik erfolgen. Insbesondere ist das Isobuten/Methanol-Verhältnis in der Synthese an das Azeotrop aus Methanol und C₄ anzupassen, welches über Kopf der den Syntheseprozess abschließenden Kolonne abgenommen wird.

Eine besondere Weiterbildung der Erfindung sieht vor, dass das Rücklaufverhältnis der den Syntheseprozess abschließenden Kolonne zwischen 0.5 und 2.0 liegt. Dieses vergleichsweise moderate Rücklaufverhältnis spart Energie, setzt aber voraus, dass das Reaktionsgemisch weniger als 1 Gew.-% Kohlenwasserstoffe mit fünf Kohlenstoffatomen enthält.

Die den Syntheseprozess abschließende Kolonne sollte mindestens 39 theoretische Trennstufen aufweisen, um die Reinigungsaufgabe zu lösen. Sie kann wahlweise als normale Destillationskolonne oder als Reaktivdestillationskolonne ausgeführt sein. Eine Reaktivdestillationskolonne ist mit Katalysatorpackungen versehen, welche in die MTBE-Synthese katalysieren.

Eine besondere Variante des Verfahrens sieht vor, dass es im Rahmen einer zeitlich begrenzten Kampagne betrieben wird und zwar in einer Anlage, welche außerhalb der Kampagne für die Herstellung von Methyl-tert.-butylether in Kraftstoffqualität genutzt wird. Vorteil davon ist, dass keine gesonderte MTBE/S Anlage benötigt wird. Es wird einfach die normalerweise für Kraftstoff-MTBE genutzte Anlage zeitlich begrenzt unter erfindungsgemäßen Bedingungen gefahren und so einen hochreinen MTBE produziert, freilich unter erhöhtem Energieaufwand. Der Wechsel zwischen der Produktion von Kraftstoff-MTBE und einer MTBE/S-Kampagne und zurück wird im Wesentlichen bewerkstelligt durch Anpassung des Massenstromverhältnisses aus Sumpfstrom der den Syntheseprozess abschließenden Kolonne zu dem Massenstrom des in diese Kolonne zulaufenden, verunreinigtem MTBE. Ggf. wird auch das Methanol/Isobuten-Verhältnis in der Synthese angepasst.

Üblicherweise enthält das rohe Reaktionsgemisch aus der Synthese als Verunreinigung zusätzlich noch mindestens eine der folgenden Substanzen: Kohlenwasserstoffe mit fünf Kohlenstoffatomen, Dimethylether (DME), tert.-Butanol (TBA), Methyl-sec.-butylether (MSBE), Wasser. DME und MSBE sind höher als MTBE siedende Substanzen. Wasser, Alkohol, C5-Kohlenwasserstoffe sind gegenüber dem MTBE Leichtsieder. Ein solches rohes Reaktionsgemisch wird in nach der Lehre der Erfindung in die die Synthese abschließende Kolonne gefahren.

Ferner kann das Reaktionsgemisch als Verunreinigung zusätzlich noch mindestens eine der folgenden Substanzen enthalten: n-Buten, n-Butan, Isobutan, 1.3-Butadien, Pentene, Pentane, Pentadien.

Die vorliegende Erfindung kann nicht nur als Verfahren zur Herstellung für hochreinen MTBE, sondern auch als Verfahren zur Reinigung von verunreinigtem MTBE unter Erhalt von hochreinen MTBE aufgefasst werden.

Mithin ist ein Verfahren zur Reinigung von verunreinigtem Methyl-tert.-butylether zwecks Erhalt von hochreinem Methyl-tert.-butylether, mit den folgenden Schritten ein zweiter Gegenstand der Erfindung:
a) Bereitstellen von verunreinigtem Methyl-tert.-butylether, welcher Methyl-tert.-butylether und als Verunreinigungen zumindest Kohlenwasserstoffe mit vier Kohlenstoffatomen, Methanol und höher als Methyl-tert.-butylether siedende Substanzen enthält;
b) Durchführen eines Reinigungsprozesses, in Zuge dessen die Verunreinigungen des verunreinigtem Methyl-tert.-butylether nahezu vollständig destillativ entfernt werden, wodurch hochreiner Methyl-tert.-butylether erhalten wird;
c) wobei der Reinigungsprozess unter Verwendung von genau zwei Destillationskolonnen durchgeführt wird, nämlich einer primären Kolonne und einer sekundären Kolonne;
d) wobei die Menge an Methanol in dem verunreinigtem Methyl-tert.-butylether kleiner ist als die Menge an Methanol in einem Azeotrop, welches aus Methanol und Kohlenwasserstoffen mit vier Kohlenstoffatomen in der primären Kolonne gebildet und über deren Kopf abgezogen wird,
e) und wobei das Sumpfprodukt der primären Kolonne in der sekundären Kolonne destillativ getrennt wird in hochreinen Methyl-tert.-butylether, welcher vom Kopf der sekundären Kolonne abgezogen wird, und in eine Fraktion enthaltend höher als Methyl-tert.-butylether siedende Substanzen.

Die primäre Kolonne des Reinigungsverfahrens entspricht der die Synthese abschließende Kolonne bei dem Herstellungsverfahren.

Der verunreinigte MTBE, welcher für zwecks Aufreinigung bereitgestellt wird, kann beispielsweise der Spezifikation I gehorchen:

| | |
|---|---|
| Methyl-tert.-butylether: | von 98.0 Gew.-% bis 99.7 Gew.-%; |
| Methyl-sec.-butylether: | von 0.01 Gew-% bis 0.2 Gew.-%; |
| tert.-Butanol: | von 0.006 Gew-% bis 0.8 Gew.-%; |
| Methanol: | von 0.05 Gew-% bis 1 Gew.-%; |
| n-Buten + Isobuten + Butadien: | von 0.05 Gew-% bis 1 Gew.-%; |
| Pentane + Pentene + Pentadien: | von 0 Gew.-ppm bis 500 Gew.-ppm; |
| Diisobuten: | von 0.005 Gew-% bis 0.2 Gew.-%; |
| Wasser: | von 0 Gew.-ppm bis 500 Gew.-ppm; |
| nicht bestimmbare Substanzen: | von 0 Gew.-ppm bis 100 Gew.-ppm; |

Die enthaltenden Komponenten ergänzen sich selbstverständlich zu 100 Gew.-%.

Das von der primären Kolonne abgezogene Sumpfprodukt entspricht vorzugsweise der Spezifikation II:

| | |
|---|---|
| Methyl-tert.-butylether: | von 99.0 Gew.-% bis 99.7 Gew.-%; |
| Methyl-sec.-butylether: | von 0.05 Gew-% bis 0.25 Gew.-%; |
| tert.-Butanol: | von 0.05 Gew-% bis 0.1 Gew.-%; |
| Methanol: | von 0 Gew.-ppm bis 10 Gew.-ppm; |
| n-Buten + Isobuten + Butadien: | von 0 Gew.-ppm bis 10 Gew.-ppm; |
| Pentane + Pentene + Pentadien: | von 0 Gew.-ppm bis 500 Gew.-ppm; |
| Diisobuten: | von 10 Gew.-ppm bis 100 Gew.-ppm; |
| Wasser: | von 0 Gew.-ppm bis 500 Gew.-ppm; |
| nicht bestimmbare Substanzen: | von 0 Gew.-ppm bis 100 Gew.-ppm; |

Die enthaltenden Komponenten ergänzen sich selbstverständlich zu 100 Gew.-%.

Das von der sekundären Kolonne abgezogene Kopfprodukt (also der hochreine MTBE) entspricht vorzugsweise der Spezifikation III:

| | |
|---|---|
| Methyl-tert.-butylether: | von 99.9 Gew.-% bis 99.99 Gew.-%; |
| Methyl-sec.-butylether: | von 0.008 Gew-% bis 0.01 Gew.-%; |
| tert.-Butanol: | von 0.004 Gew-% bis 0.006 Gew.-%; |
| Methanol: | von 0.00015 Gew-% bis 0.00025 Gew.-%; |
| n-Buten + Isobuten + Butadien: | von 0.00035 Gew-% bis 0.00045 Gew.-%; |
| Pentane + Pentene + Pentadien: | von 0 Gew.-ppm bis 10 Gew.-ppm; |
| Diisobuten: | von 0 Gew-% bis 0.00035 Gew.-%; |
| Wasser: | von 0 Gew.-ppm bis 100 Gew.-ppm; |
| nicht bestimmbare Substanzen: | von 0 Gew.-ppm bis 10 Gew.-ppm; |

Die enthaltenden Komponenten ergänzen sich selbstverständlich zu 100 Gew.-%.

Vom Sumpf der zweiten Kolonne wird MTBE abgezogen, der mit Hochsiedern verunreinigt ist. Die Verunreinigungen werden in der Praxis nicht so groß sein, sodass die Spezifikation von Kraftstoff-MTBE noch eingehalten wird. Das Sumpfprodukt der zweiten Kolonne kann mithin als Kraftstoff-MTBE vermarktet werden.

Die Erfindung soll nun anhand eines Beispiels näher erläutert werden. Hierfür zeigt:

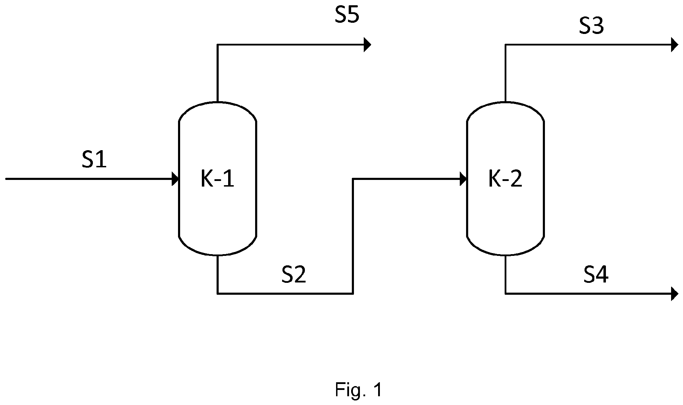

- Figur 1:: Kolonnenverschaltung des erfindungsgemäßen Verfahrens

Für die Herstellung des hochreinen MTBE werden genau zwei Destillationskolonnen benötigt, eine primäre Destillationskolonne K1 und eine sekundäre Destillationskolonne K2.

Die primäre Destillationskolonne K1 wird mit verunreinigtem Methyl-tert-butylether S1 beschickt. Der verunreinigte MTBE stammt wahlweise unmittelbar aus einer ortsnahen MTBE-Synthese (nicht dargestellt) oder wird als Kraftstoff-MTBE aus der Ferne angeliefert. Im Falle einer vorhandenen MTBE-Synthese stellt die primäre Destillationskolonne K1 die den Syntheseprozess abschließende Kolonne dar. Bei dem verunreinigten MTBE S1 handelt es sich dann um das rohe Reaktionsgemisch der Synthese. Sofern keine eigene MTBE-Synthese betrieben wird, kann auch Kraftstoff-MTBE zugekauft werden, welcher dann als verunreinigter MTBE S1 der primären Kolonne K1 aufgegeben wird.

In der primären Kolonne K1 werden über Kopf die Leichtsieder S5 aus dem verunreinigten MTBE abgetrennt. Dies sind vor allem Methanol und Kohlenwasserstoffe mit vier Kohlenstoffatomen, insbesondere Isobuten. Erfindungsgemäß wird ein verunreinigter MTBE S1 erzeugt bzw. zugekauft oder anderweitig bereitgestellt, der nur so viel Methanol enthält, wie aus dem Azeotrop aus Methanol und C₄ sich über Kopf der primären Kolonne K1 (i.e. Strom S5) abziehen lässt. Konkret wird das Methanol / Isobuten-Verhältnis in der MTBE-Synthese entsprechend an das in S5 enthaltene Azeotrop angepasst. Das Kopfprodukt S5 der primären Kolonne K1 enthält auch etwaige weitere Leichtsieder wie Wasser, TBA oder C₅.

Das Sumpfprodukt S2 der primären Kolonne K1 enthält MTBE und die höher als MTBE siedenden Verunreinigungen. Um diese zu entfernen, wird das Sumpfprodukt S2 der primären Kolonne K1 in die sekundäre Kolonne K2 gefahren.

Vom Sumpf der sekundären Kolonne K2 wird die Fraktion S4 abgezogen, welche die höher als MTBE siedenden Verunreinigungen (z. B. MSBE oder DME) bilden. In der Regel wird man das Sumpfprodukt S4 der sekundären Kolonne K2 als Kraftstoff-MTBE vermarkten. Am Kopf der sekundären Kolonne K2 wird indes das Zielprodukt S3, nämlich hochreiner MTBE gewonnen.

Dass das hier dargestellte Destillationskonzept mit genau zwei Kolonnen funktioniert, belegt eine Prozesssimulation:
Um Destillationsschnitte von Stoffen mittels der Prozesssimulation zu berechnen, ist ein thermodynamisches Modell erforderlich. Die vorgestellten Ergebnisse basieren auf der Stoffdatenmethode NRTL-RK.

Die Abkürzung NRTL steht hier für Non-Random-Two-Liquid. Hierbei handelt es sich um ein thermodynamisches Modell zur Berechnung der Aktivitätskoeffizienten der flüssigen Phase, wie es von Renon und Prausnitz beschrieben wurde:
H. Renon and J.M. Prausnitz: Local Compositions in Thermodynamic Excess Functions for Liquid Mixtures. AIChE J., Vol. 14, No. 1, (1968), pp. 135 - 144

Die Dampfphase wird mit einer Zustandsgleichung beschrieben; in diesem Fall mit der Redlich-Kwong (RK) Zustandsgleichung, welche die Dampfphase bis zu mittleren Drücken hinreichend genau beschreibt.
O. Redlich and J.N.S. Kwong: On the Thermodynamics of Solutions V. An Equation-ofstate. Fugacities of Gaseous Solutions. Chem. Rev., Vol. 44, (1949), pp. 223 - 244.

Die Eigenschaften der Reinstoffe (wie z. B. Dampfdruck, Dichte usw.) werden durch entsprechende Gleichungen beschrieben. Die erforderlichen Parameter sind entweder den Datenbanken eines Simulationsprogramms entnommen oder wurden durch Anpassung an experimentelle Daten erhalten. Das Verhalten von Mehrkomponentensystemen wird beim NRTL-Modell aus Informationen der binären Systeme vorausberechnet. Die NRTL-Parameter für die jeweiligen binären Paarungen werden an experimentell bestimmte Phasengleichgewichte (evtl. auch an Mischungswärmen oder Aktivitätskoeffizienten in unendlicher Verdünnung) angepasst. Als Prozesssimulationswerkzeug wurde das Programm AspenPlus von AspenTechnologies Inc., Bedford, Massachusetts, USA in der Version 7.3 verwendet. Die verwendeten Stoffdatenparameter stammen aus der Datenbank des Programms AspenPlus (Version 7.3). Teilweise wurden die binären Wechselwirkungsparameter unter Verwendung der Stoffdatensammlung Detherm (Dechema) und eigener Messdaten angepasst.

Dieses Vorgehen ist anerkannte Praxis zur Entwicklung und Optimierung von chemischen Prozessen.

In dem vorliegenden Beispiel wird der einer MTBE-Synthese entstammender Strom S1 der primären Kolonne K-1 (also der die Synthese abschließenden Kolonne) zugeführt. Die primäre Kolonne K-1 wird bei einem absoluten Druck von 6.5*10⁵ Pa betrieben. Es wurde ein Druckverlust von 200*10² Pa über die gesamte Kolonne angenommen. Die theoretische Stufenzahl beträgt 40 und die Kolonne wird mit einem Totalkondensator und einem Kettle-Verdampfer simuliert. Als Zulaufstufe (Feedstufe) wurde Stufe 21 gewählt. Das massenbezogene Rücklaufverhältnis wurde auf 0.71 und das massenbezogene Verhältnis von Sumpfstrommenge zu Feedstrommenge auf 0.466 eingestellt. Der Destillatstrom wird als Strom S5 bezeichnet. Mit diesen Einstellung für die Kolonnensimulation und dem hinterlegten Stoffdatenmodell ergibt sich der Sumpfstrom S2 wie in Tabelle 2 angegeben.

Strom S2 enthält nur noch sehr geringen Mengen an Methanol, Wasser, DME, und Kohlenwasserstoffe mit vier und fünf Kohlenstoffatomen. Diese bezüglich MTBE leichter siedenden Komponenten können in der primären Kolonne K-1 über Kopf abgetrennt werden.

Durch Anpassung des Verhältnis aus Sumpfstrom S2 der primären Kolonne K2 zu dem Feedstrom S1 (verunreinigter MTBE) auf diese Kolonne kann zwischen normaler Produktion von technischem MTBE und einer Kampagne zur Produktion für hochreinen MTBE umgeschaltet werden: Sofern MTBE/S produziert werden soll, beträgt das Verhältnis der Massenströme S2/S1 etwa 0.66. Soll technischer MTBE aus dem verunreinigtem MTBE hergestellt werden, wird das Verhältnis der Massenströme S2/S1 auf etwa 0.47 reduziert. Das Rücklaufverhältnis der primären Kolonne K1 kann dann im Wesentlichen unverändert bleiben. Durch Anpassung des Massenverhältnisses S2/S1 kann also eine zeitlich begrenzte Kampagne für die Herstellung von hochreinem MTBE eingeleitet und auch wieder beendet werden. Bei Produktion von MTBE/S steigt der Energiebedarf der ersten Destillationskolonne um lediglich 1.5 %.
Der Sumpfstrom S2 der primären Kolonne K-1 wird der sekundären Destillationskolonne K-2 zugeführt. Diese wurde wie folgt simuliert: Die sekundäre Kolonne K-2 wird bei einem absoluten Druck von 4*10⁵ Pa betrieben. Es wurde ein Druckverlust von 200*10² Pa über die gesamte Kolonne angenommen. Die theoretische Stufenzahl beträgt 30 und die Kolonne wird mit einem Totalkondensator und einem Kettle-Verdampfer simuliert. Als Zulaufstufe (Feedstufe) wurde Stufe 20 gewählt. Das massenbezogene Rücklaufverhältnis wurde auf 1.8 und das massenbezogene Verhältnis von Destillatmengenstrom zu Feedstrommenge auf 0.7 eingestellt. Der Strom S3 entspricht dem Destillat, wo das Zielprodukt MTBE-S mit der erforderlichen Reinheit anfällt. Der Sumpfstrom S4 kann als technisches MTBE vertrieben werden. Die Zusammensetzung, Druck und Temperatur der erwähnten Ströme S1 bis S5 ist in Tabelle 2 niedergelegt.

**Tabelle 2: Zusammensetzung Temperatur und Druck der Ströme S1 bis S5**

| **Stromname** | **S1** | **S2** | **S3** | **S4** | **S5** |
|---|---|---|---|---|---|
| Temperatur [°C] | 39.3 | 127.5 | 104.2 | 106.5 | 55.0 |
| Druck [10⁵ Pa] | 8.5 | 6.7 | 4.0 | 4.2 | 6.5 |

| Zusammensetzung [Gewichtsanteile | | | | | |
|---|---|---|---|---|---|
| DME | 725 ppm | < 1 ppm | < 1 ppm | < 1 ppm | 1357 ppm |
| BUTA-ISO | 0.031 | < 1 ppm | < 1 ppm | < 1 ppm | 0.058 |
| BUTE-ISO | 0.006 | < 1 ppm | < 1 ppm | < 1 ppm | 0.011 |
| BDIEN-13 | 0.002 | < 1 ppm | < 1 ppm | < 1 ppm | 0.005 |
| BUTE-1 | 0.227 | < 1 ppm | < 1 ppm | < 1 ppm | 0.424 |
| BUTA-N | 0.105 | < 1 ppm | < 1 ppm | < 1 ppm | 0.197 |
| BUTE-TRA | 0.089 | < 1 ppm | < 1 ppm | < 1 ppm | 0.167 |
| BUTE-CIS | 0.049 | < 1 ppm | < 1 ppm | < 1 ppm | 0.091 |
| PNTA-NEO | 0.003 | < 1 ppm | < 1 ppm | < 1 ppm | 0.006 |
| PNTA-ISO | 810 ppm | 99 ppm | 142 ppm | < 1 ppm | 1429 ppm |
| PNTA-N | < 1 ppm | < 1 ppm | < 1 ppm | < 1 ppm | < 1 ppm |
| MEOH | 0.021 | < 1 ppm | < 1 ppm | < 1 ppm | 0.039 |
| MTBE | 0.463 | 0.995 | 1.000 | 0.985 | < 1 ppm |
| MSBE | 93 ppm | 199 ppm | 132 ppm | 354 ppm | < 1 ppm |
| TBA | 1333 ppm | 0.003 | 224 ppm | 0.009 | < 1 ppm |
| H2O | 284 ppm | < 1 ppm | < 1 ppm | < 1 ppm | 531 ppm |
| DIBTE | 723 ppm | 1552 ppm | < 1 ppm | 0.005 | < 1 ppm |

### Verzeichnis der Bezugszeichen und Abkürzungen

- C4: Kohlenwasserstoffe mit vier Kohlenstoffatomen
- C5: Kohlenwasserstoffe mit fünf Kohlenstoffatomen
- K1: primäre Destillationskolonne
- K2: sekundäre Destillationskolonne
- S1: verunreinigter Methyl.-tert.-butylether
- S2: Sumpfprodukt der primären Kolonne
- S3: hochreiner MTBE
- S4: Verunreinigungen, höher als MTBE siedend (Schwersieder)
- S5: Verunreinigungen, niedriger als MTBE siedend (Leichtsieder)
- DME: Dimethylether
- BUTA-ISO: Isobutan
- BUTE-ISO: Isobuten
- BDIEN-13: 1,3-Butadien
- BUTE-11-: Buten
- BUTA-N: n-Butan
- BUTE-TRA: trans-2-Buten
- BUTE-CIS: cis-2-Buten
- PNTA-NEO: Neopentan (2,2-Dimethylpropan)
- PNTA-ISO: Isopentan
- PNTA-N: n-Pentan
- MEOH: Methanol
- MTBE: Methyl-tert.-butylether (2-methoxy-2-methylpropane)
- MSBE: Methyl-sec.-butylether
- TBA: tert.-Butanol
- H2O: Wasser
- DIBTE: Diisobuten

## Patentansprüche

1. Verfahren zur Herstellung von hochreinem Methyl-tert.-butylether, bei welchem in einem Syntheseprozess Isobuten mit Methanol zu einem Reaktionsgemisch umgesetzt wird, welches Methyl-tert.-butylether und als Verunreinigungen zumindest Kohlenwasserstoffe mit vier Kohlenstoffatomen, Methanol und höher als Methyl-tert.-butylether siedende Substanzen enthält, und bei welchem sodann unter nicht alleiniger Verwendung einer den Syntheseprozess abschließenden Kolonne in einem Reinigungsprozess die Verunreinigungen des Reaktionsgemisches nahezu vollständig destillativ entfernt werden, wodurch hochreiner Methyl-tert.-butylether erhalten wird,
**dadurch gekennzeichnet,**
**dass** Syntheseprozess und Reinigungsprozess so aufeinander abgestimmt sind, dass die Menge an Methanol in dem Reaktionsgemisch kleiner ist als die Menge an Methanol in einem Azeotrop, welches aus Methanol und Kohlenwasserstoffen mit vier Kohlenstoffatomen in der den Syntheseprozess abschließenden Kolonne gebildet und über deren Kopf abgezogen wird,
und **dass** das Sumpfprodukt der den Syntheseprozess abschließenden Kolonne in einer zweiten, im Reinigungsprozess verwendeten Kolonne destillativ getrennt wird in hochreinen Methyl-tert.-butylether, welcher vom Kopf der zweiten im Reinigungsprozess verwendeten Kolonne abgezogen wird, und in eine Fraktion enthaltend höher als Methyl-tert.-butylether siedende Substanzen.

2. Verfahren nach Anspruch 1, **dadurch gekennzeichnet, dass** das Rücklaufverhältnis der den Syntheseprozess abschließenden Kolonne zwischen 0.5 und 2.0 liegt, und dass das Reaktionsgemisch weniger als 1 Gew-% Kohlenwasserstoffe mit fünf Kohlenstoffatomen enthält.

3. Verfahren nach Anspruch 1 oder 2, **dadurch gekennzeichnet, dass** die den Syntheseprozess abschließende Kolonne mindestens 39 theoretische Trennstufen aufweist.

4. Verfahren nach Anspruch 2 oder 3, **dadurch gekennzeichnet, dass** es im Rahmen einer zeitlich begrenzten Kampagne betrieben wird, in einer Anlage, welche außerhalb der Kampagne für die Herstellung von Methyl-tert.-butylether in Kraftstoffqualität genutzt wird.

5. Verfahren nach Anspruch 1 oder nach einem der Ansprüche 2 bis 4, **dadurch gekennzeichnet, dass** das Reaktionsgemisch als Verunreinigung zusätzlich noch mindestens eine der folgenden Substanzen enthält: Kohlenwasserstoffe mit fünf Kohlenstoffatomen, Dimethylether, tert.-Butanol, Methyl-sec.-butylether, Wasser.

6. Verfahren nach Anspruch 1 oder nach einem der Ansprüche 2 bis 5, **dadurch gekennzeichnet, dass** das Reaktionsgemisch als Verunreinigung zusätzlich noch mindestens eine der folgenden Substanzen enthält: n-Buten, n-Butan, Isobutan, 1.3-Butadien, Pentene, Pentane, Pentadien.

7. Verfahren zur Reinigung von verunreinigtem Methyl-tert.-butylether zwecks Erhalt von hochreinem Methyl-tert.-butylether, mit den folgenden Schritten:
a) Bereitstellen von verunreinigtem Methyl-tert.-butylether, welcher Methyl-tert.-butylether und als Verunreinigungen zumindest Kohlenwasserstoffe mit vier Kohlenstoffatomen, Methanol und höher als Methyl-tert.-butylether siedende Substanzen enthält;
b) Durchführen eines Reinigungsprozesses, in Zuge dessen die Verunreinigungen des verunreinigtem Methyl-tert.-butylether nahezu vollständig destillativ entfernt werden, wodurch hochreiner Methyl-tert.-butylether erhalten wird;
**dadurch gekennzeichnet**,
c) dass der Reinigungsprozess unter Verwendung von genau zwei Destillationskolonnen durchgeführt wird, nämlich einer primären Kolonne und einer sekundären Kolonne;
d) dass die Menge an Methanol in dem verunreinigtem Methyl-tert.-butylether kleiner ist als die Menge an Methanol in einem Azeotrop, welches aus Methanol und Kohlenwasserstoffen mit vier Kohlenstoffatomen in der primären Kolonne gebildet und über deren Kopf abgezogen wird,
e) und dass das Sumpfprodukt der primären Kolonne in der sekundären Kolonne destillativ getrennt wird in hochreinen Methyl-tert.-butylether, welcher vom Kopf der sekundären Kolonne abgezogen wird, und in eine Fraktion enthaltend höher als Methyl-tert.-butylether siedende Substanzen.

8. Verfahren nach Anspruch 7, **dadurch gekennzeichnet, dass** der verunreinigte Methyl-tert.-butylether mit folgender Spezifikation I bereitgestellt wird:
| | |
|---|---|
| Methyl-tert.-butylether: | von 98.0 Gew.-% bis 99.7 Gew.-%; |
| Methyl-sec.-butylether: | von 0.01 Gew-% bis 0.2 Gew.-%; |
| tert.-Butanol: | von 0.006 Gew-% bis 0.8 Gew.-%; |
| Methanol: | von 0.05 Gew-% bis 1 Gew.-%; |
| n-Buten + Isobuten + Butadien: | von 0.05 Gew-% bis 1 Gew.-%; |
| Pentane + Pentene + Pentadien: | von 0 Gew.-ppm bis 500 Gew.-ppm; |
| Diisobuten: | von 0.005 Gew-% bis 0.2 Gew.-%; |
| Wasser: | von 0 Gew.-ppm bis 500 Gew.-ppm; |
| nicht bestimmbare Substanzen: | von 0 Gew.-ppm bis 100 Gew.-ppm; |
wobei sich die enthaltenden Komponenten zu 100 Gew.-% ergänzen.

9. Verfahren nach Anspruch 7 oder 8, **dadurch gekennzeichnet, dass** das Sumpfprodukt der primären Kolonne folgender Spezifikation II gehorcht:
| | |
|---|---|
| Methyl-tert.-butylether: | von 99.0 Gew.-% bis 99.7 Gew.-%; |
| Methyl-sec.-butylether: | von 0.05 Gew-% bis 0.25 Gew.-%; |
| tert.-Butanol: | von 0.05 Gew-% bis 0.1 Gew.-%; |
| Methanol: | von 0 Gew.-ppm bis 10 Gew.-ppm; |
| n-Buten + Isobuten + Butadien: | von 0 Gew.-ppm bis 10 Gew.-ppm; |
| Pentane + Pentene + Pentadien: | von 0 Gew.-ppm bis 500 Gew.-ppm; |
| Diisobuten: | von 10 Gew.-ppm bis 100 Gew.-ppm; |
| Wasser: | von 0 Gew.-ppm bis 500 Gew.-ppm; |
| nicht bestimmbare Substanzen: | von 0 Gew.-ppm bis 100 Gew.-ppm; |
wobei sich die enthaltenden Komponenten zu 100 Gew.-% ergänzen.

10. Verfahren nach Anspruch 7 oder 8 oder 9, **dadurch gekennzeichnet, dass** der am Kopf der sekundären Kolonne hochreiner Methyl-tert.-butylether mit folgender Spezifikation III abgezogen wird:
| | |
|---|---|
| Methyl-tert.-butylether: | von 99.9 Gew.-% bis 99.99 Gew.-%; |
| Methyl-sec.-butylether: | von 0.008 Gew-% bis 0.01 Gew.-%; |
| tert.-Butanol: | von 0.004 Gew-% bis 0.006 Gew.-%; |
| Methanol: | von 0.00015 Gew-% bis 0.00025 Gew.-%; |
| n-Buten + Isobuten + Butadien: | von 0.00035 Gew-% bis 0.00045 Gew.-%; |
| Pentane + Pentene + Pentadien: | von 0 Gew.-ppm bis 10 Gew.-ppm; |
| Diisobuten: | von 0 Gew-% bis 0.00035 Gew.-%; |
| Wasser: | von 0 Gew.-ppm bis 100 Gew.-ppm; |
| nicht bestimmbare Substanzen: | von 0 Gew.-ppm bis 10 Gew.-ppm; |
wobei sich die enthaltenden Komponenten zu 100 Gew.-% ergänzen.
